# EUROPÄISCHE PATENTSCHRIFT

(11) **EP 1 261 285 B1**
(45) Veröffentlichungstag und Bekanntmachung des Hinweises auf die Patenterteilung: **09.04.2008**
(21) Anmeldenummer: 00958088.7
(22) Anmeldetag: 19.09.2000
(51) Int. Cl.: A61B 17/16

(54) **KUPPLUNGSVORRICHTUNG FÜR INSTRUMENTEN-BESTANDTEILE**
COUPLING DEVICE FOR INSTRUMENT PARTS
DISPOSITIF D'ACCOUPLEMENT POUR PARTIES CONSTITUTIVES D'INSTRUMENTS

(30) Priorität: 06.03.2000 WO PCT/CH00/00126
(43) Veröffentlichungstag der Anmeldung: 04.12.2002
(73) Patentinhaber: Synthes GmbH, 4436 Oberdorf (CH)
(72) Erfinder: STEIGER, Peter, CH-3360 Herzogenbuchsee (CH)
(74) Vertreter: Lusuardi, Werther
(86) Internationale Anmeldenummer: PCT/CH2000/000507
(87) Internationale Veröffentlichungsnummer: WO 2001/066024

(56) Entgegenhaltungen:
- WO-A-88/03786
- DE-C- 19 813 699
- US-A- 5 720 749

## Beschreibung

Die Erfindung bezieht sich auf eine Vorrichtung zur Kupplung von Instrumenten, insbesondere von chirurgischen Instrumenten und/oder Implantaten gemäss dem Oberbegriff des Patentanspruchs 1.

Eine Kupplung zur Verbindung eines Räumwerkzeuges an eine Antriebsmaschine ist aus der US 5,720,749 RUPP bekannt. Dieses bekannte Räumwerkzeug dient insbesondere für die Markraumaufbohrung in Knochen und umfasst im wesentlichen einen lösbar an eine flexible, rotativ antreibbare Welle kuppelbaren Schneidkopf. Die Kupplungsteile bestehen aus einer rohrförmigen Buchse am Schneidwerkzeug und einem ebenfalls rohrförmigen, in die Bohrung der Buchse einschiebbaren Zapfen an der flexiblen Welle. Buchse und Zapfen sind im wesentlichen als Hohlzylinder ausgestaltet, wobei die äussere Mantelfläche des Zapfens und die innere Mantelfläche der Buchse auf einem Teil ihrer Länge mit zwei parallelen, zueinander korrespondierenden Flächen versehen sind. Diese Flächen enden am Zapfen vor dem kupplungsseitigen Ende, so dass an diesem Ende des Zapfens zwei Nocken entstehen. Zudem ist die Wand des Zapfens mit axialen Schlitzen versehen, so dass federnde Zungen entstehen. Beim Einschieben des Zapfens in die Buchse werden die beiden mit Nocken versehenen Zungen radial zusammengepresst, so lange bis die Nocken bei vollständig eingeschobenem Zapfen in entsprechende periphere, die Wand der Buchse durchdringende Schlitze einschnappen. Damit ist der Zapfen rotativ und axial in der Buchse festgehalten. Zum Entfernen des Schneidkopfes werden die federnden Zungen des Zapfens von ausserhalb der Buchse durch die peripheren Schlitze hindurch zusammengepresst, so dass die Nocken aus diesen Schlitzen ausrasten und der Zapfen aus der Buchse herausgezogen werden kann. Nachteilig an dieser bekannten Vorrichtung ist die Notwendigkeit, zur Demontage des Schneidkopfes über eine spezielle Vorrichtung zum Zusammenpressen der federnden Zungen durch die peripheren Schlitze hindurch verfügen zu müssen. Zudem ist die Verbindung zwischen Welle und Zapfen eine Pressverbindung, wodurch ein in einer Bohrung im Zapfen geführter Führungs- oder Kirschnerdraht nur unter erheblichem Reibungsverlust rotierbar ist.

Hier will die Erfindung Abhilfe schaffen. Der Erfindung liegt die Aufgabe zugrunde, eine Kupplung zu schaffen, welche eine Montage und eine Demontage der aneinanderkuppelbaren Teile ohne Hilfsinstrumente oder -vorrichtungen ermöglicht. Zudem ist die Kupplung so beschaffen, dass im zusammengefügten Zustand in beiden Richtungen eine axiale und rotative Blockierung entsteht.

Die Erfindung löst die gestellte Aufgabe mit einer Vorrichtung zur Kupplung von Instrumenten, insbesondere von chirurgischen Instrumenten und/oder Implantaten, welche die Merkmale des Anspruchs 1 aufweist.

Weitere vorteilhafte Ausgestaltungen der Erfindung sind in den abhängigen Ansprüchen gekennzeichnet.

Die erfindungsgemässe Vorrichtung zur Kupplung von chirurgischen Instrumenten und/oder Implantaten umfasst einen Verbindungszapfen und eine Buchse, welche eine Bohrung zur Aufnahme des Verbindungszapfens aufweist, wobei die Mantelfläche des Verbindungszapfens in einem Abstand A vom vorderen Ende beginnend und sich von dort über eine Länge L parallel zur Längsachse erstreckend n Flächen und einen polygonalen Querschnitt aufweist und die Bohrung der Buchse dazu korrespondierend n Wandflächen umfasst, so dass der Verbindungszapfen in der Bohrung spielfrei und um die Längsachse verdrehfest aufnehmbar ist. Der Verbindungszapfen umfasst mindestens einen radialen, vom vorderen Ende bis zu einer Tiefe T eindringenden Schlitz, so dass vom vorderen Ende bis zur Tiefe T radial federnde Zungen entstehen. Zudem umfasst die Mantelfläche gegen das vordere Ende des Verbindungszapfens mindestens einen, radial über den Verbindungszapfen oder über mindestens eine der Flächen vorstehenden Nocken. Die Zungen sind radial so weit komprimierbar, dass der Verbindungszapfen bei komprimierten Zungen vom hinteren Ende der Buchse her in den mit n Wandflächen versehenen Teil der Bohrung einführbar ist und in einem in der Bohrung angebrachten, zu dem Nocken korrespondierenden Hinterstich einschnappbar ist, so dass eine axiale Blockierung des Verbindungszapfens in der Buchse erreichbar ist. Nocken und Hinterstich umfassen Mittel, welche gestatten, dass bei einer auf den Verbindungszapfen wirkenden, gegen das hintere Ende der Buchse gerichteten Zugkraft von mindestens der Grösse X die Zungen so weit radial komprimierbar sind, dass der Nocken aus dem Hinterstich ausrastet und der Verbindungszapfen aus der Bohrung herausziehbar ist. Vorzugsweise beträgt die zum Komprimieren der Zungen notwendige Zugkraft 1 bis 50 N.

Die Mittel bestehen darin, dass die Ausgestaltung der Nocken in einer Ausführungsform in axialer Richtung konvex ist, oder dass in weiteren Ausführungsformen der/die Nocken axial keilartig mit einem Winkel α in mindestens eine der Flächen am Verbindungszapfen münden.

Der Winkel α kann in allen der oben erwähnten Ausführungsformen zwischen 15° und 85°, vorzugsweise zwischen 25° und 35° betragen.

Der Verbindungszapfen kann in einer Ausführungsform der erfindungsgemässen Vorrichtung mit einer um die Längsachse rotierbaren Antriebswelle verbunden sein, während an der Buchse beispielsweise ein Räumwerkzeug angebracht ist.

In der bevorzugten Ausführungsform der erfindungsgemässen Vorrichtung hat die Mantelfläche des Verbindungszapfens die Form eines Kreiszylinders und die Nocken werden durch Anbringen von n ebenen Flächen auf der Mantelfläche gebildet, wobei die Flächen gegenüber der Mantelfläche in radialer Richtung teilweise vertieft sind. Der Hinterstich ist als Hinterdrehung ausgebildet und grenzt an den mit den Wandflächen versehenen Teil der Bohrung. Die Hinterdrehung mündet gegen das hintere Ende mit einem ersten Innenkonus in die Wandflächen der Bohrung, wobei der halbe Konuswinkel des ersten Innenkonus vorzugsweise dem Winkel α entspricht.

In einer weiteren Ausführungsform der erfindungsgemässen Vorrichtung ist die gegen das vordere Ende gerichtete Stirnfläche der Hinterdrehung orthogonal zur Längsachse ausgerichtet und als ebene Stirnfläche ausgebildet. Bei eingeführtem Verbindungszapfen steht das vordere Ende des Verbindungszapfens an der orthogonal zur Längsachse stehenden Stirnfläche der Hinterdrehung an.

In wiederum einer anderen Ausführungsform der erfindungsgemässen Vorrichtung ist der mindestens eine Nocken am vorderen Ende parallel zur Längsachse keilartig ausgestaltet und schliesst gegen das vordere Ende hin mit der Längsachse einen Winkel β ein. Der Winkel β kann in allen der oben erwähnten Ausführungsformen zwischen 15° und 85°, vorzugsweise zwischen 25° und 35° betragen.

In einer weiteren Ausführungsform der erfindungsgemässen Vorrichtung mündet die Hinterdrehung gegen das vordere Ende gerichtet mit einem zweiten Innenkonus in die Bohrung der Buchse, wobei der halbe Konuswinkel des zweiten Innenkonus vorzugsweise dem Winkel β entspricht. Bei eingeführtem Verbindungszapfen stehen die keilartigen Nocken am vorderen Ende des Verbindungszapfens am zweiten Innenkonus der Hinterdrehung an.

Die axialen Längen von Nocken und Hinterdrehung stimmen vorzugsweise so überein, dass bei in der Hinterdrehung eingeschnappten Nocken in beiden Richtungen eine axiale Blockierung des Verbindungszapfens in der Buchse entsteht.

In wiederum einer anderen Ausführungsform der erfindungsgemässen Vorrichtung weist der Verbindungszapfen eine zur Längsachse konzentrische Bohrung auf, so dass konzentrisch zur Längsachse ein Führungs- oder Kirschnerdraht durch Verbindungszapfen und Buchse bringbar ist.

Die durch die Erfindung erreichten Vorteile sind im wesentlichen darin zu sehen, dass dank der erfindungsgemässen Vorrichtung zwei Bestandteile eines chirurgischen Instrumentes auf einfache Weise und ohne weitere Hilfsmittel zusammengekuppelt und auch wieder voneinander getrennt werden können. Zudem ist im eingekuppelten Zustand ein Führungs- oder Kirschnerdraht durch den Verbindungszapfen und die Buchse führbar, ohne dass bei einer Rotation der Vorrichtung relativ zum Führungs- oder Kirschnerdraht Reibungsverluste entstehen. Die Vorrichtung gestattet im eingekuppelten Zustand eine Verbindung, welche kein Verdrehen und keine axiale Verschiebung des Verbindungszapfens relativ zur Buchse zulässt.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch die bevorzugte Ausführungsform der erfindungsgemässen Vorrichtung;
Fig. 2 einen Querschnitt der in Fig. 1 dargestellten Ausführungsform der erfindungsgemässen Vorrichtung;
Fig. 3 einen Längsschnitt durch eine weitere Ausführungsform der erfindungsgemässen Vorrichtung; und
Fig. 4 eine perspektivische Ansicht des Verbindungszapfens mit einem Schnitt durch die Buchse einer weiteren Ausführungsform der erfindungsgemässen Vorrichtung.

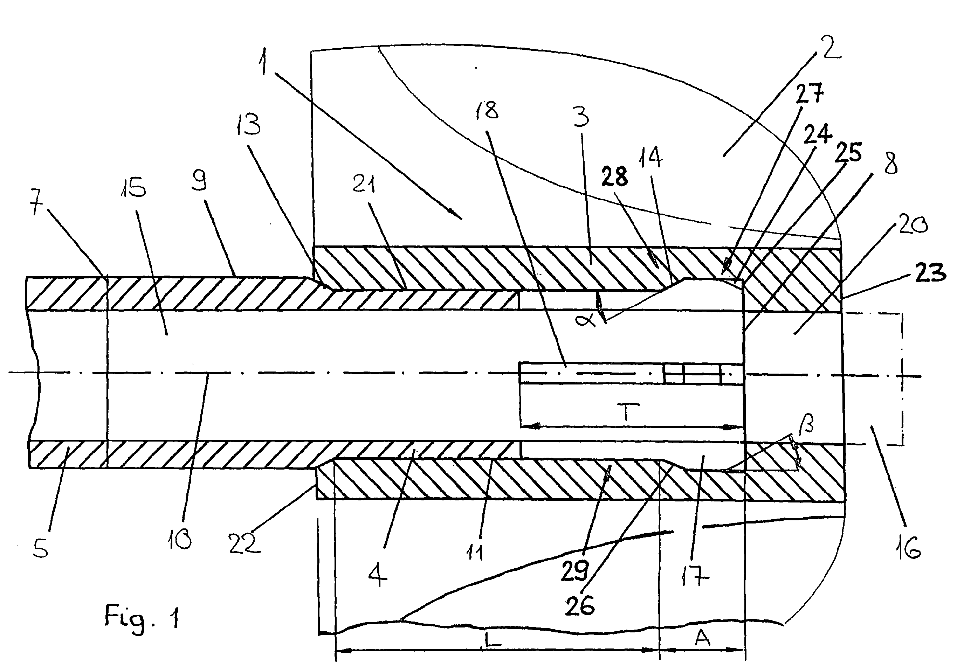

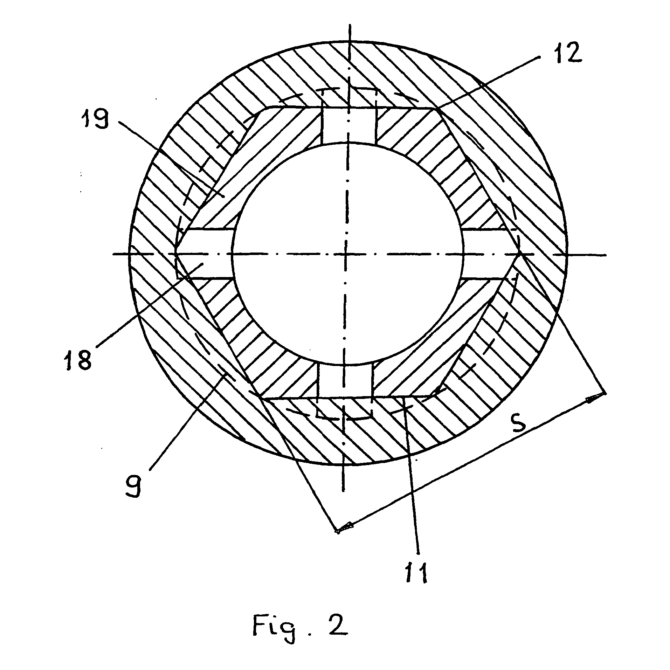

In Fig. 1 und 2 sind Schnitte durch die bevorzugte Ausführungsform der erfindungsgemässen Kupplung 1 dargestellt. Mittels der Kupplung 1 werden eine beispielsweise mit dem Schneidkopf 2 eines Räumwerkzeuges ausgestattete Buchse 3 und der Verbindungszapfen 4 einer flexiblen Antriebswelle 5 miteinander lösbar verbunden, wobei im eingekuppelten Zustand Buchse 3 und Verbindungszapfen 4 relativ zueinander rotativ und axial blockiert sind. Der Verbindungszapfen 4 ist hohlzylindrisch ausgebildet und weist eine Längsachse 10, wellenseitig ein hinteres Ende 7 und von der Antriebswelle 5 entfernt ein vorderes Ende 8 auf. Die axiale Bohrung 15 im Verbindungszapfen 4 dient zur Aufnahme eines Führungs- oder Kirschnerdrahtes 16. Die äussere Mantelfläche 9 des Verbindungszapfens 4 ist im wesentlichen kreiszylindrisch, weist einen Durchmesser D auf und ist mit sechs ebenen Flächen 11 versehen, wobei jeweils zwei dieser Flächen 11 zur Längsachse 10 symmetrisch sind. In der hier beschriebenen Ausführungsform der erfindungsgemässen Vorrichtung ist die Schlüsselweite S zwischen zwei parallelen Flächen 11 kleiner als der Durchmesser D des Verbindungszapfens 4 und so bemessen, dass zwischen zwei benachbarten Flächen 11 abgerundete, auf der äusseren Mantelfläche 9 liegende Kanten 12 entstehen. Ebenfalls sind am Verbindungszapfen 4 vom vorderen Ende 8 her parallel zur Längsachse 10 vier symmetrisch auf dem Umfang des Verbindungszapfens 4 verteilte Schlitze 18 bis zu einer Tiefe T angebracht, so dass sich vier radial federbare Zungen 19 bilden. Die Flächen 11 haben in axialer Richtung eine Länge L, welche sich ab einem Abstand A vom vorderen Ende 8 gegen das hintere Ende 7 erstreckt, so dass an dem sich vom vorderen Ende 8 bis zum Abstand A erstreckenden Teil der äusseren Mantelfläche 9 Nocken 17 gebildet werden. Die quer zur Längsachse 10 entstehenden Übergänge 13 und 14 zwischen den Flächen 11 und der äusseren Mantelfläche 9 sind keilartig, wobei zwischen dem vorderen, in die Nocken 17 mündenden Übergang 14 und der Längsachse 10 ein Winkel α von 30° eingeschlossen wird. 6. Die Nocken 17 sind am vorderen Ende 8 parallel zur Längsachse 10 keilartig ausgestaltet, so dass die Nocken 17 am vorderen Ende 8 mit der Längsachse 10 einen Winkel β von 30° einschliessen. Die Buchse 3 hat ein hinteres wellenseitiges Ende 22, ein vorderes Ende 23 und weist korrespondierend zu den Flächen 11 am Verbindungszapfen 4 in ihrer zur Längsachse 10 fluchtenden Bohrung 20 maximal auf der Länge L sechs ebene Wandflächen 21 auf, so dass der in die Bohrung 20 der Buchse 3 eingefügte Verbindungszapfen 4 gegen Rotation um die Längsachse 10 gesichert ist. An die Wandflächen 21 gegen das vordere Ende 23 angrenzend ist die Bohrung 20 der Buchse 3 mit einer Hinterdrehung 24 ausgestattet, wobei die gegen das vordere Ende 23 gerichtete Stirnfläche 25 der Hinterdrehung 24 orthogonal zur Längsachse 10 steht während die Hinterdrehung 24 gegen das hintere Ende 22 mit einem ersten Innenkonus 26 in die Wandflächen 21 mündet. Der halbe Konuswinkel des Innenkonus 26 entspricht dem Winkel α an den Übergängen 14 der Nocken 17. Die Wandflächen 21 münden parallel zur Längsachse 10 keilartig sich erweiternd in das hintere Ende 22 der Buchse 3, wobei der Keilwinkel vorzugsweise dem Winkel β entspricht und ebenfalls 30° beträgt. Durch diese Ausgestaltung von Buchse 3 und Verbindungszapfen 4 kann dieser vom hinteren Ende 22 der Buchse 3 her in die Bohrung 20 eingeschoben werden, wobei die radial über die Wandflächen 21 vorstehenden Nocken 17 durch radiales Zusammenpressen der federnden Zungen 19 in die Buchse 3 einführbar sind. Wird der Verbindungszapfen 4 so weit in die Bohrung 20 eingeführt, dass die Nocken 17 in den Bereich der Hinterdrehung 24 kommen, federn die Zungen 19 aus und die Nocken 17 schnappen in die Hinterdrehung 24 ein. Im eingeschnappten Zustand des Verbindungszapfens 4 in der Buchse 3 wird durch das Anliegen des vorderen Endes 8 auf der Stirnfläche 25 der Hinterdrehung 24 ein axialer Anschlag gegen das vordere Ende 23 der Buchse 3 gebildet, während durch den Innenkonus 26 und die Übergänge 14 der Verbindungszapfen 4 axial gegen das hintere Ende 22 der Buchse 3 blockiert ist. Entspricht die durch den Abstand A gebildete Breite der Nocken 17 der Breite der Hinterdrehung 24 so ist der Verbindungszapfen 4 im eingeschnappten Zustand gegen axiale Verschiebungen in beiden Richtung blockiert. Zudem gestatten die Keilform der Übergänge 14 am Verbindungszapfen 4 und der Innenkonus 26 an der Hinterdrehung 24, dass durch Aufbringen einer axialen Zugkraft auf den Verbindungszapfen 4 gegen das hintere Ende 22 der Buchse 3 die federnden Zungen 19 radial zusammengepresst werden, wodurch die Nocken 17 aus der Hinterdrehung 24 ausrasten können und somit der Verbindungszapfen 4 aus der Buchse 3 herausgezogen werden kann. Der Winkel α des ersten Innenkonus 26 und der Übergänge 14 ist dabei vorzugsweise so gewählt, dass zum Zusammenpressen der federnden Zungen 19 eine grössere Axialkraft aufgebracht werden muss, als bei normalem Gebrauch des Instrumentes auftritt. Zudem können die federnden Zungen 19 bei durch die Bohrung 15 im Verbindungszapfen 4 eingeführtem Führungs- oder Kirschnerdraht 16 nicht einfedern, so dass bei normalem Gebrauch der Verbindungszapfen 4 durch die Nocken 17 zusätzlich gegen das hintere Ende 22 der Buchse 3 gesichert ist.

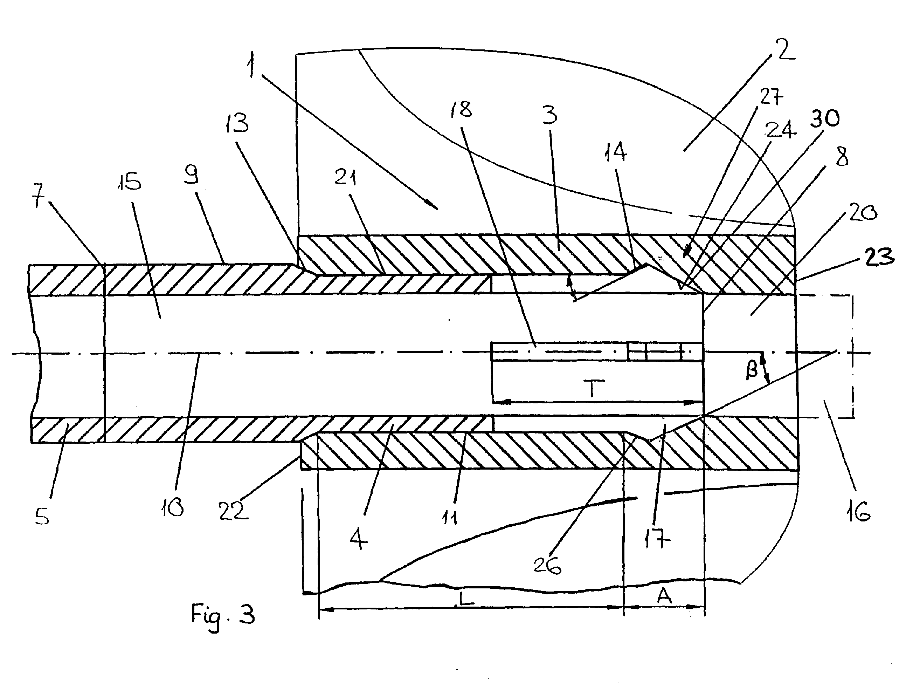

In Fig. 3 ist eine Ausführungsform der erfindungsgemässen Vorrichtung dargestellt, welche sich von der in Fig. 1 gezeigten Ausführungsform nur darin unterscheidet, dass die Hinterdrehung 24 gegen das vordere Ende 23 der Buchse 3 gerichtet mit einem zweiten Innenkonus 30 in die Bohrung 20 mündet, wobei der halbe Konuswinkel des zweiten Innenkonus 26 dem Winkel β entspricht.

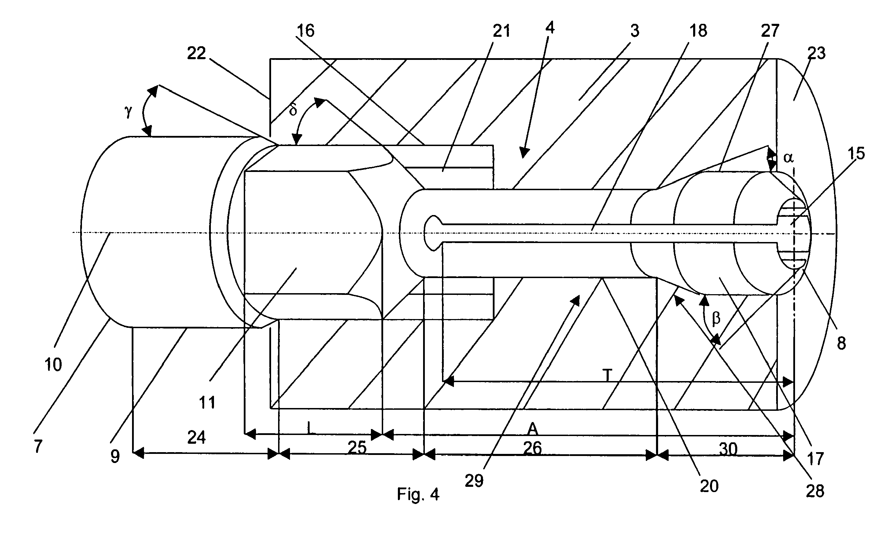

Fig. 4 zeigt eine Ausführungsform der erfindungsgemässen Vorrichtung, welche sich von der in den Fig. 1 und 2 dargestellten Ausführungsform darin unterscheidet, dass der Verbindungszapfen 4 zwischen dem hinteren Ende 7 und dem vorderen Ende 8 axial aneinandergrenzend vier koaxiale Segmente 24; 25; 26 und 30 umfasst. Das an das hintere Ende 7 angrenzende, erste Segment 24 ist kreiszylindrisch ausgestaltet und ist am hinteren Ende 7 mit einer Antriebswelle (nicht gezeichnet) verbindbar. Gegen das vordere Ende 8 gerichtet, ist das zweite Segment 25 an das erste Segment 24 angefügt. Das zweite Segment 25 ist prismatisch mit sechseckigem Querschnitt ausgestaltet, wobei das Eckmass des Sechskantes kleiner als der Aussendurchmesser des ersten Segmentes 24 ist und der Übergang vom ersten Segment 24 zum zweiten Segment 25 konisch mit einem halben Konuswinkel δ = 45° ausgestaltet ist. Weiter gegen das vordere Ende 8 an das zweite Segment 25 angrenzend ist ein kreiszylindrisches, drittes Segment 26 angefügt, dessen Aussendurchmesser kleiner als die Schlüsselweite des sechskantigen, zweiten Segmentes 25 ist. Auch der Übergang zwischen dem zweiten Segment 25 und dem dritten Segment 26 ist konisch mit einem halben Konuswinkel δ von 45°. An das dritte Segment 26 angefügt und bis zum vorderen Ende 8 reichend ist das vierte Segment 30, welches einen grösseren Aussendurchmesser als das dritte Segment 26 aufweist, einerseits konisch mit einem halben Konuswinkel α von 30° in das dritte Segment 26 mündet und andererseits konisch mit einem halben Konuswinkel β von 30° am vorderen Ende 7 abschliesst. Der Verbindungszapfen 4 umfasst vom vorderen Ende 8 bis zu einer Tiefe T eine koaxiale Bohrung 15 und drei ebenfalls vom vorderen Ende 8 bis zu einer Tiefe T reichende, die Wand des Verbindungszapfens 4 zwischen der Bohrung 15 und der äusseren Mantelfläche 9 parallel zur Längsachse 10 trennende Schlitze 18. Mittels der Schlitze 18 wird eine radiale Kompressibilität des Verbindungszapfens 4 auf der Tiefe T erreicht und der Verbindungszapfen 4 in diesem axialen Abschnitt als radial elastisches Spannteil 29 ausgestaltet. Durch den grösseren Aussendurchmesser des vierten Segmentes 30 gegenüber dem dritten Segment 26 werden durch die ausgekröpfte und geschlitzte Wand des Verbindungszapfens 4 drei radial gegenüber dem dritten Segment 26 vorstehende Nocken 17 gebildet. Komplementär zum Verbindungszapfen 4 ist die Buchse 3 ausgestaltet, welche beispielsweise am vorderen Ende 23 mit einem chirurgischen Instrument (nicht gezeichnet) verbindbar ist. Die Bohrung 20 weist gegen das hintere Ende 22 der Buchse 3 gerichtet einen Innensechskant 16 auf, welcher beim zusammenfügen der Buchse 3 mit dem zweiten Segment 25 des Verbindungszapfens 4 eine bezüglich der Längsachse 10 verdrehfeste Verbindung bildet. Dieser Innensechskant 16 mündet konisch komplementär zum konischen Übergang zwischen dem ersten Segment 24 und dem zweiten Segment 25 in die Stirnfläche am hinteren Ende 22 der Buchse 3. Gegen das vordere Ende 23 der Buchse 3 umfasst die Bohrung 20 einen kreiszylindrischen Hinterstich 27 mit einem den Hinterstich 27 gegenüber der Bohrung 20 abgrenzenden kegelstumpfförmigen Teil, welcher zum konischen Übergang zwischen dem dritten Segment 26 und dem vierten Segment 30 komplementär konisch in die Bohrung 20 mündet. Beim Einführen des vorderen Endes 8 des Verbindungszapfens 4 in die Bohrung 20 der Buchse 3 wird das radial elastische Spannteil 29 des Verbindungszapfens 4 radial komprimiert, so dass die Nocken 17 durch den kreiszylindrischen Mittelteil der Bohrung 20 axial gegen das_vordere Ende 23 der Buchse 3 schiebbar sind bis die Nocken den Hinterstich 27 erreichen, wo das elastische Spannteil 29 wieder in seine unkomprimierte Ausgangslage zurückfedern kann und die Nocken 17 in den Hinterstich 27 einrasten. Damit ist der Verbindungszapfen 4 bezüglich der Längsachse 10 verdrehfest und gegen axiale Verschiebung gesichert in der Buchse 3 gelagert. Beim Aufbringen einer genügend grossen Zugkraft auf den Verbindungszapfen 4 in Richtung des hinteren Endes 23 der Buchse 3 werden die Nocken 17 durch die komplementär konischen Übergänge zwischen einerseits dem dritten und vierten Segment 26; 30 am Verbindungszapfen 4 und andererseits zwischen der Bohrung 20 und dem Hinterstich 27 radial komprimiert und der Verbindungszapfen 4 ist axial gegen das hintere Ende 23 der Buchse 3 aus dieser herausziehbar.

## Patentansprüche

1. Vorrichtung zur Kupplung von Instrumenten und/oder Implantaten mit
A) einem longitudinalen Verbindungszapfen (4), welcher ein hinteres Ende (7), ein vorderes Ende (8), eine Längsachse (10) und eine Mantelfläche (9) umfasst;
B) einer Buchse (3), welche ein hinteres Ende (22), ein vorderes Ende (23) und eine Bohrung (20) zur Aufnahme des Verbindungszapfens (4) koaxial zur Längsachse (10) aufweist, wobei
C) die Mantelfläche (9) des Verbindungszapfens (4) parallel zur Längsachse (10) in einem Abstand (A) vom vorderen Ende (8) beginnend und sich von dort über eine Länge L erstreckend n Flächen (11) aufweist und die Bohrung (20) der Buchse (3) dazu korrespondierend mindestens n Wandflächen (21) umfasst, so dass der Verbindungszapfen (4) in der Bohrung (20) um die Längsachse (10) verdrehfest aufnehmbar ist;
D) der Verbindungszapfen (4) koaxial zur Längsachse (10) vom vorderen Ende (8) bis mindestens zu einer Tiefe T eine Bohrung (15) und mindestens einen radialen Schlitz (18) umfasst, so dass der Verbindungszapfen vom vorderen Ende (8) bis zur Tiefe T ein radial elastisch komprimierbares Spannteil (29) aufweist;
E) der Verbindungszapfen (4) mindestens einen, radial vorstehenden Nocken (17) umfasst;
G) die Bohrung (20) am vorderen Ende (23) der Buchse (3) zu dem mindestens einen Nocken (17) korrespondierend einen Hinterstich (27) umfasst, worin der Nocken (17) einschnappbar ist, so dass der Verbindungszapfen (4) in der Buchse (3) axial blockierbar ist, wobei
H) Nocken (17) und Hinterstich (27) Mittel (28) umfassen, wodurch bei einer auf den Verbindungszapfen (4) wirkenden, gegen das hintere Ende (22) der Buchse (3) gerichteten Zugkraft von mindestens der Grösse X der Spannteil (29) radial komprimierbar ist, so dass der Nocken (17) aus dem Hinterstich (27) ausrastet und der Verbindungszapfen (4) aus der Bohrung (20) herausziehbar ist,
**dadurch gekennzeichnet, dass**
I) der Verbindungszapfen (4) im Längenabschnitt mit der Länge L einen polygonalen Querschnitt aufweist.

2. Vorrichtung nach Anspruch 1, **dadurch gekennzeichnet, dass** das hintere Ende (7) des Verbindungszapfens mit einer Antriebswelle (5) verbindbar ist.

3. Vorrichtung nach Anspruch 1 oder 2, **dadurch gekennzeichnet, dass** die Buchse (3) mit einem Räumwerkzeug (2) verbindbar ist.

4. Vorrichtung nach einem der Ansprüche 1 bis 3, **dadurch gekennzeichnet, dass** die Mittel (28) darin bestehen, dass der mindestens eine Nocken (17) in axialer Richtung konvex ausgestaltet ist.

5. Vorrichtung nach einem der Ansprüche 1 bis 3, **dadurch gekennzeichnet, dass** die Mittel (28) darin bestehen, dass der mindestens eine Nocken (17) parallel zur Längsachse (10) keilartig mit einem Winkel α in eine der Flächen (11) mündet.

6. Vorrichtung nach einem der Ansprüche 1 bis 5, **dadurch gekennzeichnet, dass** der mindestens eine Nocken (17) am vorderen Ende (8) parallel zur Längsachse (10) keilartig ausgestaltet ist, so dass der Nocken (17) am vorderen Ende (8) mit der Längsachse (10) einen Winkel β einschliesst.

7. Vorrichtung nach einem der Ansprüche 1 bis 6, **dadurch gekennzeichnet, dass** der Verbindungszapfen (4) n Nocken (17) umfasst.

8. Vorrichtung nach Anspruch 5 oder einem der Ansprüche 6 bis 7, in Verbindung mit Anspruch 5, **dadurch gekennzeichnet, dass** der Winkel α zwischen 15° und 85° beträgt.

9. Vorrichtung nach Anspruch 8, **dadurch gekennzeichnet, dass** der Winkel α zwischen 25° und 35° beträgt.

10. Vorrichtung nach einem der Ansprüche 1 bis 9, **dadurch gekennzeichnet, dass** die Zugkraft 1 bis 50 N beträgt.

11. Vorrichtung nach Anspruch 6 oder einem der Ansprüche 7 bis 10 in Verbindung mit Anspruch 6, **dadurch gekennzeichnet, dass** der Winkel β zwischen 15° und 85° beträgt.

12. Vorrichtung nach Anspruch 11, **dadurch gekennzeichnet, dass** der Winkel β zwischen 25° und 35° beträgt.

13. Vorrichtung nach einem der Ansprüche 1 bis 12, **dadurch gekennzeichnet, dass** der Hinterstich (27) eine Hinterdrehung (24) ist.

14. Vorrichtung nach Anspruch 13, **dadurch gekennzeichnet, dass** die Hinterdrehung mit einem ersten Innenkonus (26) in die mindestens eine Wandfläche (21) mündet.

15. Vorrichtung nach Anspruch 14 in Verbindung mit Anspruch 5, **dadurch gekennzeichnet, dass** der halbe Konuswinkel des ersten Innenkonus (26) dem Winkel α entspricht.

16. Vorrichtung nach einem der Ansprüche 13 bis 15, **dadurch gekennzeichnet, dass** die Hinterdrehung (24) eine gegen das vordere Ende (23) gerichtete, orthogonal zur Längsachse (10) stehende ebene Stirnfläche (25) aufweist.

17. Vorrichtung nach Anspruch 14 oder 15 in Verbindung mit Anspruch 6, **dadurch gekennzeichnet, dass** die Hinterdrehung (24) gegen das vordere Ende (23) gerichtet mit einem zweiten Innenkonus (30) in die Bohrung (20) mündet.

18. Vorrichtung nach Anspruch 17, **dadurch gekennzeichnet, dass** der halbe Konuswinkel des zweiten Innenkonus (26) dem Winkel β entspricht.

19. Vorrichtung nach einem der Ansprüche 1 bis 18, **dadurch gekennzeichnet, dass** die Bohrung (15) des Verbindungszapfens (4) eine zur Längsachse (10) konzentrische, durchgehende Bohrung (15) ist, so dass konzentrisch zur Längsachse (10) ein Führungs- oder Kirschnerdraht (16) durch Verbindungszapfen (4) und Buchse (3) bringbar ist.

20. Vorrichtung nach einem der Ansprüche 1 bis 19, **dadurch gekennzeichnet, dass** der Verbindungszapfen (4) im Längenabschnitt mit der Länge L einen hexagonalen Querschnitt aufweist.

21. Vorrichtung nach einem der Ansprüche 1 bis 20, **dadurch gekennzeichnet, dass** die Wandflächen (21) axial keilförmig sich erweiternd in das hintere Ende (22) der Buchse (3) münden.

22. Vorrichtung nach Anspruch 21, **dadurch gekennzeichnet, dass** der Keilwinkel dem Winkel β entspricht.

23. Vorrichtung nach einem der Ansprüche 1 bis 22, **dadurch gekennzeichnet, dass** der Verbindungszapfen (4) gegen das vordere Ende (8) axial an die n Flächen (11) anschliessend mindestens einen, radial über mindestens eine der n Flächen (11) vorstehenden Nocken (17) umfasst.

24. Vorrichtung nach Anspruch 23, **dadurch gekennzeichnet, dass** die Bohrung (20) gegen das vordere Ende (23) der Buchse (3) axial an die mindestens n Wandflächen (21) angrenzend und zu dem mindestens einen Nocken (17) korrespondierend einen Hinterstich (27) umfasst, worin der Nocken (17) einschnappbar ist, so dass der Verbindungszapfen (4) in der Buchse (3) axial blockierbar ist.

## Claims

1. An apparatus for coupling instruments and/or implants including
A) a longitudinal male connector (4) which comprises a rear end (7), a front end (8), a longitudinal axis (10), and a peripheral surface (9);
B) a hollow coupling shank (3) which comprises a rear end (22), a front end (23), and a bore (20) for receiving the male connector (4) coaxially to the longitudinal axis (10),
C) the peripheral surface (9) of the male connector (4) having n flats (11) parallel to the longitudinal axis (10) beginning at a distance (A) from the front end (8) and extending therefrom over a length L, and the bore (20) of the hollow coupling shank (3) comprising at least n wall surfaces (21) corresponding to said flats, so that the male connector (4) can be inserted into the bore (20) in a manner which is resistant to twisting about the longitudinal axis (10);
D) the male connector (4) comprising a bore (15) extending coaxially to the longitudinal axis (10) from the front end (8) to at least a depth T and at least one radial slot (18), so that the male connector is provided with a radially elastically compressible tension member (29) extending from the front end (8) to the depth T;
E) the male connector (4) comprising at least one radially protruding lug (17);
F) the bore (20) comprising at the front end (23) of the hollow coupling shank (3) and corresponding to the at least one lug (17) a recess (27) with which the lug (17) may engage so that the male connector (4) can be axially blocked in the hollow coupling shank (3), whereby
G) said lug (17) and said recess (27) comprise means (28) which permit that on applying on the male connector (4) a tensile force of at least the amount X which is directed towards the rear end (22) of the hollow coupling shank (3), the tension member (29) may be radially compressed so that the lug (17) will disengage from the recess (27) and the male connector (4) can be removed from the bore (20),
**characterised in that**
H) the male connector (4) in its longitudinal segment with the length L has a polygonal cross section.

2. An apparatus as claimed in claim 1, **characterised in that** the rear end (7) of the male connector is connectable to a drive shaft (5).

3. An apparatus as claimed in claim 1 or 2, **characterised in that** said hollow coupling shank (3) is connectable to a reamer (2).

4. An apparatus as claimed in any of the claims 1 to 3, **characterised in that** said means (28) consist in an axially convex shape of the at least one lug (17).

5. An apparatus as claimed in any of the claims 1 to 3, **characterised in that** said means (28) consist in the fact that the at least one lug (17) leads parallel to the longitudinal axis (10) in a wedge-shaped manner with an angle α into one of the flats (11).

6. An apparatus as claimed in any of the claims 1 to 5, **characterised in that** the at least one lug (17) is parallel to the longitudinal axis (10) wedge-shapedly configured at the front end (8) so that the lug (17) at the front end (8) forms an angle β relative to the longitudinal axis (10).

7. An apparatus as claimed in any of the claims 1 to 6, **characterised in that** said male connector (4) comprises n lugs (17).

8. An apparatus as claimed in claim 5 or in any of the claims 6 to 7 in connection with claim 5, **characterised in that** said angle α is between 15° and 85°.

9. An apparatus as claimed in claim 8, **characterised in that** said angle α is between 25° and 35°.

10. An apparatus as claimed in any of the claims 1 to 9, **characterised in that** said tensile force is between 1 and 50 N.

11. An apparatus as claimed in claim 6 or in any of the claims 7 to 10 in connection with claim 6, **characterised in that** the angle β is between 15° and 85°.

12. An apparatus as claimed in claim 11, **characterised in that** the angle β is between 25° and 35°.

13. An apparatus as claimed in any of the claims 1 to 12, **characterised in that** the recess (27) has the form of a relieved portion (24).

14. An apparatus as claimed in claim 13, **characterised in that** the relieved portion has a first inner cone (26) leading to the wall surface (21).

15. An apparatus as claimed in claim 14 in connection with claim 5, **characterised in that** half the cone angle of said first inner cone (26) corresponds to the angle α.

16. An apparatus as claimed in any of the claims 13 to 15, **characterised in that** the relieved portion (24) has an end face (25) which faces the front end (23) and is oriented orthogonally to the longitudinal axis (10).

17. An apparatus as claimed in claim 14 or 15 in connection with claim 6, **characterised in that** the relieved portion (24) has a second inner cone (30) situated towards the front end (23) and leading to the bore (20).

18. An apparatus as claimed in claim 17, **characterised in that** half the cone angle of said second inner cone (26) corresponds to the angle β.

19. An apparatus as claimed in any of the claims 1 to 18, **characterised in that** the bore (15) of the male connector (4) is a bore (15) extending concentrically to the longitudinal axis (10) through its entire length, so that a guide wire or Kirschner wire (16) may be passed concentrically to the longitudinal axis (10) through both the male connector (4) and the hollow coupling shank (3).

20. An apparatus as claimed in claim 1, **characterised in that** the male connector (4) in its longitudinal segment with the length L has a hexagonal cross section.

21. An apparatus as claimed in any of the claims 1 to 20, **characterised in that** the wall surfaces (21) lead into the rear end (22) of the hollow coupling shank (3) in an axially wedge-shaped enlarging manner.

22. An apparatus as claimed in claim 21, **characterised in that** the cone angle corresponds to the angle β.

23. An apparatus as claimed in any of the claims 1 to 22, **characterised in that** the male connector (4) comprises towards the front end (8) and axially adjacent to the n flats (11) at least one lug (17) protruding radially over at least one of the n flats (11).

24. An apparatus as claimed in claim 23, **characterised in that** the bore (20) comprises towards the front end (22) of the hollow coupling shank (3) axial adjacent to the at least n wall surfaces (21) and corresponding to the at least one lug (17) a recess (27) with which the lug (17) may engage so that the male connector (4) can be axially blocked in the hollow coupling shank (3).

## Revendications

1. Dispositif d'accouplement d'instruments et/ou d'implants, comprenant
A) une broche de liaison longitudinale (4) qui comprend une extrémité arrière (7), une extrémité avant (8), un axe longitudinal (10) et une enveloppe externe (9) ;
B) une douille (3) qui présente une extrémité arrière (22), une extrémité avant (23) et un alésage (20) destiné à loger la broche de liaison (4) coaxialement à l'axe longitudinal (10), dans lequel
C) l'enveloppe externe (9) de la broche de liaison (4) présente, parallèlement à l'axe longitudinal (10), n surfaces (11) commençant à une distance (A) en partant de l'extrémité avant (8) et s'étendant sur une longueur L, et l'alésage (20) de la douille (3) comprend au moins n parois latérales (21) correspondant à celles-ci, de sorte que la broche de liaison (4) peut être logée dans l'alésage (20) sans pouvoir tourner autour de l'axe longitudinal (10) ;
D) la broche de liaison (4) comprend, coaxialement à l'axe longitudinal (10), de l'extrémité avant (8) et au moins jusqu'à une profondeur T, un alésage (15) et au moins une fente radiale (18), de sorte que la broche de liaison présente, de l'extrémité avant (8) jusqu'à la profondeur T, un élément de serrage (29) radialement élastiquement compressible;
E) la broche de liaison (4) comprend au moins un mentonnet dépassant radialement (17) ;
G) l'alésage (20) comprend, au niveau de l'extrémité avant (23) de la douille (3), une dépouille (27), correspondant à au moins un mentonnet (17), dans laquelle le mentonnet (17) peut être encliqueté, de sorte que la broche de liaison (4) peut être bloquée axialement dans la douille (3), dans lequel
H) le mentonnet (17) et la dépouille (27) comprennent des moyens (28) grâce auxquels l'élément de serrage (29) est comprimable radialement sous l'effet d'une force de traction d'au moins la dimension X exercée sur la broche de liaison (4), dirigée vers l'extrémité arrière (22) de la douille (3), de sorte que le mentonnet (17) se désencliquette de la dépouille (27) et la broche de liaison (4) peut être retirée de l'alésage (20),
**caractérisée en ce que**
I) la broche de liaison (4) présente, sur le segment longitudinal de longueur L, une section polygonale.

2. Dispositif selon la revendication 1, **caractérisé en ce que** l'extrémité arrière (7) de la broche de liaison peut être reliée à un arbre d'entraînement (5).

3. Dispositif selon la revendication 1 ou 2, **caractérisé en ce que** la douille (3) peut être reliée à un outil de brochage (2).

4. Dispositif selon l'une quelconque des revendications 1 à 3, **caractérisé en ce que** les moyens (28) consistent à réaliser le au moins un mentonnet (17) sous forme convexe dans le sens axial.

5. Dispositif selon l'une quelconque des revendications 1 à 3, **caractérisé en ce que** les moyens (28) consistent à faire déboucher le au moins un mentonnet (17) parallèlement à l'axe longitudinal (10) de manière cunéiforme, à un angle α, dans l'une des surfaces (11).

6. Dispositif selon l'une quelconque des revendications 1 à 5, **caractérisé en ce que** le au moins un mentonnet (17) est réalisé, au niveau de l'extrémité avant (8), parallèlement à l'axe longitudinal (10), de manière cunéiforme, de sorte que le mentonnet (17) forme un angle β au niveau de l'extrémité avant (8) avec l'axe longitudinal (10).

7. Dispositif selon l'une quelconque des revendications 1 à 6, **caractérisé en ce que** la broche de liaison (4) comprend n mentonnets (17).

8. Dispositif selon la revendication 5 ou selon l'une quelconque des revendications 6 à 7 conjointement avec la revendication 5, **caractérisé en ce que** l'angle α est compris entre 15° et 85°.

9. Dispositif selon la revendication 8, **caractérisé en ce que** l'angle α est compris entre 25° et 35°.

10. Dispositif selon l'une quelconque des revendications 1 à 9, **caractérisé en ce que** la force de traction est de 1 à 50 N.

11. Dispositif selon la revendication 6 ou selon l'une quelconque des revendications 7 à 10 conjointement avec la revendication 6, **caractérisé en ce que** l'angle β est compris entre 15° et 85°.

12. Dispositif selon la revendication 11, **caractérisé en ce que** l'angle β est compris entre 25° et 35°.

13. Dispositif selon l'une quelconque des revendications 1 à 12, **caractérisé en ce que** la dépouille (27) est un détalonnage (24).

14. Dispositif selon la revendication 13, **caractérisé en ce que** le détalonnage débouche avec un premier cône intérieur (26) dans la au moins une paroi latérale (21).

15. Dispositif selon la revendication 14 conjointement avec la revendication 5, **caractérisé en ce que** le demi-angle de conicité du premier cône intérieur (26) correspond à l'angle α.

16. Dispositif selon l'une quelconque des revendications 13 à 15, **caractérisé en ce que** le détalonnage (24) présente une face frontale plane (25) dirigée vers l'extrémité avant (23), orthogonale à l'axe longitudinal (10).

17. Dispositif selon la revendication 14 ou 15 conjointement avec la revendication 6, **caractérisé en ce que** le détalonnage (24) débouche dans la direction de l'extrémité avant (23) avec un deuxième cône intérieur (30) dans l'alésage (20).

18. Dispositif selon la revendication 17, **caractérisé en ce que** le demi-angle de conicité du deuxième cône intérieur (26) correspond à l'angle β.

19. Dispositif selon l'une quelconque des revendications 1 à 18, **caractérisé en ce que** l'alésage (15) de la broche de liaison (4) est un alésage traversant (15) concentrique à l'axe longitudinal (10), de sorte qu'une broche de guidage ou une broche de Kirschner (16) peut être insérée concentriquement à l'axe longitudinal (10) à travers la broche de liaison (4) et la douille (3).

20. Dispositif selon l'une quelconque des revendications 1 à 19, **caractérisé en ce que** la broche de liaison (4) présente, sur un segment longitudinal de longueur L, une section hexagonale.

21. Dispositif selon l'une quelconque des revendications 1 à 20, **caractérisé en ce que** les parois latérales (21) débouchent dans l'extrémité arrière (22) de la douille (3) en s'élargissant axialement de manière cunéiforme.

22. Dispositif selon la revendication 21, **caractérisé en ce que** l'angle de conicité correspond à l'angle β.

23. Dispositif selon l'une quelconque des revendications 1 à 22, **caractérisé en ce que** la broche de liaison (4) comprend au moins un mentonnet (17), qui dépasse radialement d'au moins une des n surfaces (11), axialement adjacent aux n surfaces (11) vers l'extrémité avant (8).

24. Dispositif selon la revendication 23, **caractérisé en ce que** l'alésage (20) comprend une dépouille (27), axialement adjacente aux au moins n parois latérales (21) vers l'extrémité avant (23) de la douille (3) et correspondant au au moins un mentonnet (17), dépouille dans laquelle le mentonnet (17) peut être encliqueté, de sorte que la broche de liaison (4) peut être bloquée axialement dans la douille (3).
